(19) **Europäisches Patentamt / European Patent Office / Office européen des brevets**

(11) **EP 2 111 835 B1**

(12) **EUROPEAN PATENT SPECIFICATION**

(45) Date of publication and mention of the grant of the patent:
**07.08.2013 Bulletin 2013/32**

(51) Int Cl.:
*A61F 13/49* (2006.01)    *A61F 13/15* (2006.01)
*A61F 13/53* (2006.01)    *A61F 13/534* (2006.01)

(21) Application number: **08704069.7**

(22) Date of filing: **29.01.2008**

(86) International application number:
**PCT/JP2008/051263**

(87) International publication number:
**WO 2008/093660 (07.08.2008 Gazette 2008/32)**

(54) **ABSORPTIVE ARTICLE**

SAUGFÄHIGER ARTIKEL

ARTICLE ABSORBANT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.01.2007 JP 2007018514**

(43) Date of publication of application:
**28.10.2009 Bulletin 2009/44**

(73) Proprietors:
• **The University of Tokyo**
  **Bunkyo-Ku**
  **Tokyo 113-8654 (JP)**
• **Daio Paper Corporation**
  **Ehime 799-0492 (JP)**

(72) Inventors:
• **SANADA, Hiromi**
  **Tokyo 113-8654 (JP)**

• **MIZOBUCHI, Keita**
  **Sakura-shi**
  **Tochigi 329-1411 (JP)**

(74) Representative: **Tollett, Ian et al**
  **Williams Powell**
  **Staple Court**
  **11 Staple Inn Buildings**
  **London, WC1V 7QH (GB)**

(56) References cited:
**EP-A1- 0 948 676      EP-A1- 1 627 618
WO-A1-94/01069        WO-A1-2005/012620
JP-A- 06 205 806       JP-A- 10 503 683
JP-A- 62 045 703       JP-A- 2001 046 435
JP-A- 2006 102 479**

**Description**

Technical Field

The present invention relates to absorbent articles such as disposable diapers, sanitary napkins, urine pads, and absorbent pads.

Background Art

There are well known absorbent articles such as disposable diapers, in each of which an absorber is interposed between a liquid permeable top sheet and a liquid impermeable back sheet (refer to Patent Document 1, for example). Such absorbent articles are used in contact with a wearer's skin; and thus are often consciously or unconsciously positioned under body pressure between a wearer's body and other objects, on different occasions when the wearer lies down or sits.

In particular, absorbent articles are frequently used for those who cannot get up from their beds, and especially for people requiring nursing care. In using absorbent articles for such people, the absorbent articles are continuously positioned between the wearer and the bed or mattress, under body pressure at all times.

An absorber for use in absorbent articles is generally formed in such a manner that an absorbent polymer is scattered in a fiber base material such as accumulated pulp. In formation of such an absorber, it is common practice to design the absorber in such a manner as to have a certain degree of solidity for prevention of twists or displacement after absorbing body fluids, and to hold the absorbent polymer in base fibers at body fluid absorption in terms of body fluid retention.

Patent Document 1: JP 2006-102479 A

Disclosure of the Invention

Technical Problems to be Solved

However, conventional absorbent articles hardly have the function of body pressure dispersion due to the absorbers' solidity as stated above or the like. Accordingly, when using such an absorbent article for a long time, a wearer may get tired or suffer skin troubles due to contact between the wearer's body and the absorbent article under body pressure.

For example, after discharge of body fluids such as voiding of urine, the absorbent article is high humidity inside and causes skin maceration to the wearer, whereby the wearer's skin becomes vulnerable to damage from external factors.

At that time, if the wearer receives higher body pressure at specific sites, the wearer's skin may be harmed by skin friction or disorders at the specific sites.

Meanwhile, in recent years, there have been proposed tools for preferably dispersing body pressure one after another, such as bedclothes using low-rebound materials that are intended to contribute to reducing burdens on those who cannot get up from beds and others (hereinafter, referred to as simply "body pressure dispersion tools"). However, wearers of such absorbent articles as stated above may not fully benefit from the body pressure dispersion capabilities of the body pressure dispersion tools because the absorbent articles themselves do not disperse body pressure.

Particularly, those who cannot get up from beds or those who are in wheelchairs for a long time have a problem of decubitus ulcers (also called bedsores). Decubitus ulcers are caused mainly due to a shearing force applied between relatively hard body portions in skin, such as bones or muscles, and skin surface layers.

Further, it has been pointed out in recent years that generation of a shearing force is stimulated by using absorbent articles such as disposable diapers. Specifically, when a wearer uses an absorbent article, the wearer's skin is pressed against a skin-contact surface of the absorbent article, and the absorbent article is excessively restricted in movement in a shearing direction with respect to the contact surface of the wear's skin due to the presence of the hard absorber, whereby a strong shearing force is generated between relatively hard bones and muscles in the skin and the skin surface layers, resulting in a cause of decubitus ulcers.

Accordingly, a principal object of the present invention is to provide an absorbent article that is favorably effective in body pressure dispersion, particularly in body pressure dispersion with an absorber absorbing body fluids or the like, and effective in prevention of decubitus ulcers.

Means to Solve the Problems

The present invention to solve the foregoing problems, and operations and effects thereof will be described below.

<Invention according to Claim 1>

An absorbent article having an absorber interposed between a liquid permeable top sheet and a liquid impermeable back sheet, wherein

the absorber has a layered structure in which absorbent cores each including at least a fiber base material and an absorbent polymer as constitutional elements are layered,

the absorbent polymer is scattered in the fiber base material in such a manner that the polymer swells by gelation due to liquid absorption and expands up to one surface of the fiber base material through inter-fiber gaps, thereby to form a polymer layer on the fiber base material,

absorbent polymer expanding surfaces of adjacent absorbent cores are opposed to each other, and

at least by formation of the polymer layer, the adjacent absorbent cores can move mutually in a shearing direction with the polymer layer therebetween.

(Operation and Effect)

The absorber of the present invention has a gelled polymer layer of significant thickness on adjacent surfaces of the absorbent cores at a time of liquid absorption. The polymer layer produces the effect of body pressure dispersion due to gel-specific viscosity and elasticity of the polymer, an increased allowance for displacement of the adjacent cores in the shearing direction, and the like. Further, even if the wearer's skin is pressed against the skin-contact surface of the absorbent article while the wearer uses the absorbent article, the adjacent absorbent cores are mutually movable in the shearing direction. Accordingly, no strong friction resistance is caused between the skin-contact surface and the wearer's skin, or no strong shearing force is generated between relatively hard bones and muscles in the skin and the skin surface layers, thereby preventing occurrence of decubitus ulcers.

Meanwhile, the absorbent cores may have no polymer layer on respective surfaces opposite to the adjacent surfaces. The opposite surfaces may be used to fix the absorbent cores to other members included in the absorbent article, such as the top sheet or the back sheet, whereby it can be prevented that the absorbent cores are unnecessarily twisted or displaced.

For example, if the absorbent cores have a non-basis weight layer in which a basis weight of the absorbent polymer is virtually 0 $g/m^2$, on the surfaces opposite to the absorbent polymer expanding surfaces, the gelled absorbent polymer in a damp state after liquid absorption becomes extremely less prone to expand toward the skin-contact surfaces (the top side) by the presence of the non-basis weight layer. Therefore, the damp polymer after liquid absorption is made distant from the wearer's skin, thereby providing an improved wear comfort even after liquid absorption.

<Invention according to Claim 2>

The absorbent article according to Claim 1, wherein the fiber base material is a tow fiber aggregate, an air-laid nonwoven fabric, or a synthetic fiber aggregate.

(Operation and Effect)

A tow fiber aggregate, an air-laid nonwoven fabric, and a synthetic fiber aggregate have relatively strong entanglements between fibers, and thus preferably prevent the adjacent absorbent cores from being collapsed when the absorbent cores move mutually in the shearing direction.

<Invention according to Claim 3>

The absorbent article according to Claim 1, wherein the absorbent polymer is 150 to 500 $\mu$m in average particle diameter, and

the fiber base material is an air-through nonwoven fabric with a basis weight of 20 to 50 $g/m^2$ and a thickness of 0.4 to 10 mm, which is formed by short fibers with a fiber diameter of 2 to 20 dtex and a fiber length of 20 to 70 mm.

(Operation and Effect)

According to the present invention, it is possible to use sheet-like fiber base materials that contains predetermined short fibers with adequate gaps therebetween. This makes it possible to produce absorbent cores that hardly block movement of a swollen polymer, thereby realizing easy formation of a polymer layer.

<Invention according to Claim 4>

The absorbent article according to any one of Claims 1 to 3, wherein the absorbent cores have the absorbent polymer unevenly distributed in such a manner that a basis weight thereof becomes larger with increasing proximity to the absorbent polymer expanding surfaces.

(Operation and Effect)

Since the absorbent polymer is unevenly distributed in such a manner that a basis weight thereof becomes larger with increasing proximity to the absorbent polymer expanding surfaces, the absorbent polymer is prone to expand toward the expanding surfaces at liquid absorption, thereby facilitating formation of a polymer layer. In addition, the absorbent core can be easily designed such that a smaller amount of polymer expands toward surfaces opposite to the expanding surfaces or no polymer expands toward the opposite surfaces.

<Invention according to Claim 5>

The absorbent article according to any one of Claims 1 to 4, comprising a movement auxiliary layer in which a movement auxiliary material formed by a gas, a liquid or particles, or a mixture thereof, is interposed between the adjacent absorbent cores.

(Operation and Effect)

By interposing the movement auxiliary layer formed of a gas, a liquid or particles or a mixture thereof between the adjacent absorbent cores, it is easy to move the adjacent absorbent cores in the shearing direction while maintaining a body fluid absorption capability of the absorbent cores, in particular, of the absorbent core on the non-skin-contact side. Particularly preferred examples of such a gas, a liquid and particles include air, polymer particles, gel, bead particles, urethane, and lubricating polymers.

<Invention according to Claim 6>

The absorbent article according to Claim 5, wherein the movement auxiliary layer is formed in such a manner that a movement auxiliary material is charged into a plurality of sections divided by partitioning walls bridged between the adjacent absorbent cores.

(Operation and Effect)

By bridging the partitioning walls between the adjacent absorbent cores, it is possible to restrict amounts of movement of the absorbent cores and thus prevent the absorbent cores from moving excessively in the shearing direction. In addition, since the movement auxiliary material is charged into the sections formed by the partitioning walls, it is possible to prevent a leak of the movement auxiliary material from between the adjacent absorbent cores, and thus provide the absorbent cores with a reliable movement effect and body pressure dispersion capability.

<Invention according to Claim 7>

The absorbent article according to any one of Claims 1 to 5, wherein the adjacent absorbent cores have a sheet formed of nonwoven fabric or accumulated pulp interposed therebetween.

(Operation and Effect)

Absorbed body fluids are supposed to pass through the sheet formed of nonwoven fabric or accumulated pulp when moving between the adjacent absorbent cores, which allows the body fluids to be dispersed or absorbed at the time. Therefore, for example, the body fluids may be moved in a state of being widely dispersed in the absorbent core on the skin-contact side, or may be retained between the adjacent absorbent cores for a longer time, thereby achieving expanded applicability in design of the absorber.
In addition, upon formation of a polymer layer, the swollen polymer constituting the polymer layer attaches to the sheet, whereby it is possible to block return of the swollen polymer toward the fiber base materials. This prevents the polymer layer from disappearing or becoming thinner.

Effect of the Invention

According to the present invention as described above, it is possible to provide an absorbent article that is favorably effective in body pressure dispersion, in particular, body pressure dispersion with an absorber absorbing body fluids or the like, and effective in prevention of decubitus ulcers.

Best Mode for Carrying Out the Invention

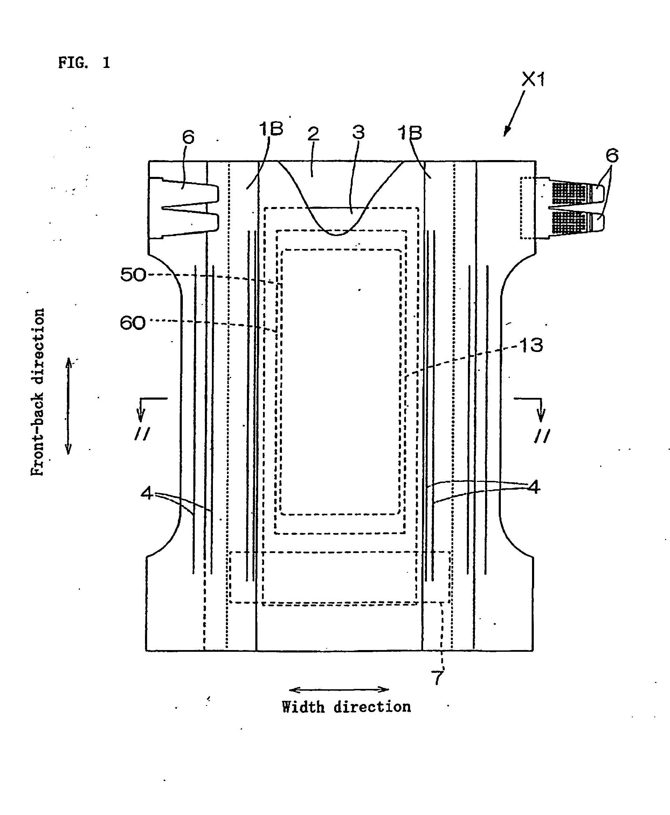

Subsequently, embodiments of the present invention will be described below in detail with reference to the drawings.
An arrangement of the present invention can be employed for body fluid absorbent articles for use in various kinds of fastening-type or underpants-type disposable diapers, sanitary napkins, absorbent pads, urine pads and the like at a wearer's body sites under body pressure. As an example, a fastening-type disposable diaper will be described below, which can be worn by engaging fastening tapes 6, 6 on both sides of a back-side waist portion to engagement sections 7 in a front-side ventral portion, as shown in FIG. 1.

<First embodiment>

A disposable diaper of a first embodiment will be described with reference to FIGs. 1 to 5.
A disposable diaper X1 of the illustrated embodiment has a layered structure in which a rectangular, or preferably sandglass-shaped absorber 3 with a certain degree of solidity is interposed between a liquid permeable top sheet 1 on a wearer's skin side and a liquid impermeable back sheet 2 that is positioned on an external side of a product and does not virtually let a liquid pass through.
In addition, the disposable diaper X1 has standing cuffs 1B that are erected on the wearer's skin side in a longitudinal direction (front-back direction) by raising resilient and elastic members 4, 4 disposed in the longitudinal direction.
The liquid permeable top sheet 1 and the liquid impermeable back sheet 2 are of the same shape as an outside planar shape of the disposable diaper X1. The two sheets each extend at front and rear ends in a front-back direction beyond front and rear ends of the absorber 3 and at both sides in a side-to-side direction beyond both side edges of the absorber 3, and the two sheets are joined at those extending sections. The two sheets may be joined by any known method, and there is no limitation on a method for the joining. For example, the sheets may be joined by hot-melt adhesion, ultrasonic sealing, heat sealing (thermal fusion bonding), heat pressing (thermal compression bonding), or a combination thereof. The fastening tapes 6, 6 are attached to areas of joining between the liquid permeable top sheet 1 and the liquid impermeable back sheet 2, particularly at both sides of the back-side waist portion. The fastening tapes 6, 6 are of a known type in which hook members of a mechanical fastener are partly arranged. The hook members are engaged with an engagement section 7 including loop members on the ventral side, whereby the diaper can be worn.
The liquid permeable top sheet 1 uses preferably a netted sheet formed by plain-woven threads of nylon, polyethylene terephthalate or the like, a film sheet material with a large number of through holes, a film sheet material of polyethylene, polypropylene, or the like.
In addition, the liquid permeable top sheet may be a nonwoven fabric sheet. Such a nonwoven fabric sheet may be formed of synthetic fibers based on olefin such as polyethylene or polypropylene, polyester, polyamide, or the like; recycled fibers such as rayon or cupra; or natural fibers such as cotton, which are processed by an appropriate processing method such as a spun lace method, spun bonding method, thermal bonding method, melt-blown method, needle punching method.
Of those nonwoven fabric sheet processing methods, the spun lace method is excellent in providing flexibility and drape property, and the thermal bonding method is excellent in achieving high bulk and softness. In addition, the liquid permeable top sheet may be a SMS nonwoven fabric sheet or a point-bonded nonwoven fabric sheet, or may be a layered-structure sheet, such as a laminated nonwoven fabric sheet in which a plastic film with through holes and a nonwoven fabric sheet are laminated.
The liquid impermeable back sheet 2 uses a sheet material having at least liquid imperviousness, formed of olefin resin sheet such as polyethylene or polypropylene. The liquid impermeable back sheet 2 may also use a microporous breathable resin sheet obtained by melting and kneading an inorganic filling agent into an olefin resin such as polyethylene or polypropylene to thereby form a sheet, and then extending the sheet in a uniaxial or biaxial direction. In addition to the foregoing, the liquid impermeable back sheet 2 may use a laminated nonwoven fabric sheet in which a nonwoven fabric is laminated on a polyethylene sheet or the like, or a nonwoven fabric sheet in which a water-proof film is interposed for virtual liquid imperviousness, or the like. More preferably, the liquid impermeable back sheet 2 use a sheet in which an outer sheet formed of a nonwoven fabric or the like is laminated on an external surface of the resin sheet for improved textures.
Meanwhile, the absorber 3 has a layered structure in which the absorbent cores 3A, 3B are layered with the absorbent polymer 31 scattered in the sheet-like fiber base materials 30. The absorber 3 is covered with a cover sheet (not shown). A material for the cover sheet only needs to have a body fluid permeability, and there is no limitation to the kind of the material. For example, the material for the cover sheet may be the same as any of those for the liquid permeable top sheet 1 listed above.

A gross basis weight of the absorbent polymer 31 in the absorber 3 ranges from 250 to 500 $g/m^2$, preferably 300 to 400 $g/m^2$, in particular preferably 330 to 340 $g/m^2$. If the gross basis weight of the absorbent polymer 31 is less than 250 $g/m^2$, the absorbent article may not sufficiently perform a body fluid absorption function. A gross basis weight exceeding 500 $g/m^2$ is too large and may lead to high production costs. The basis weight of the absorbent polymer 31 in the absorbent cores 3A, 3B may be half that of the absorber due to the two-layer structure, or the basis weight of either of the absorbent cores may be made larger than the other. For example, the basis weight of the absorbent core 3B on a side closer to the back-side layer may be increased.

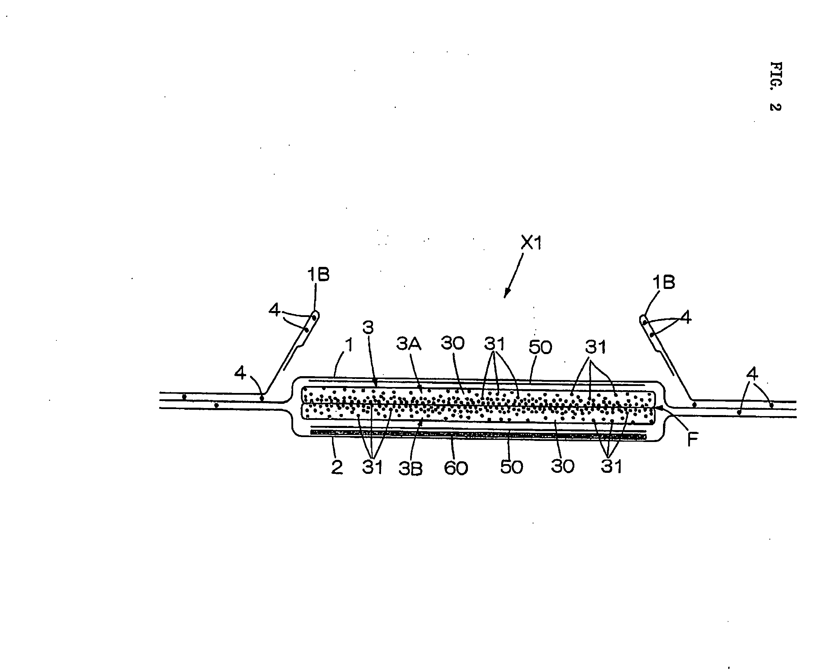

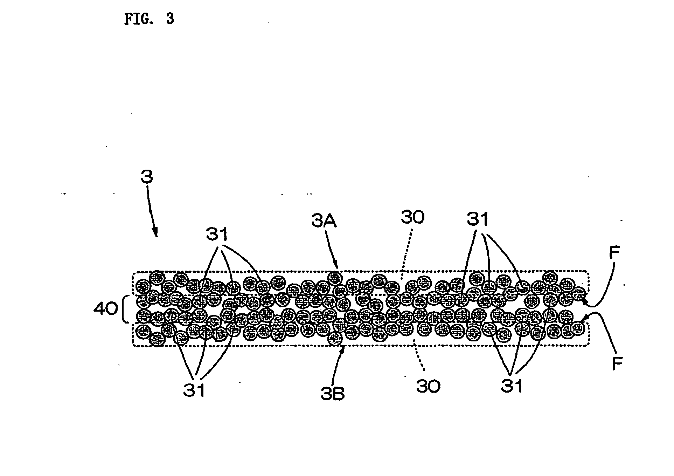

As apparent from FIGs. 2 and 3, the absorber 3 of the present invention is **characterized in that** the absorbent polymer 31 expands up to one surface of each of sheet-like fiber base materials through inter-fiber gaps in the sheet-like fiber base materials, due to swelling by gelation of the absorbent polymer 31 after liquid absorption, and the absorbent cores 3A, 3B forming a polymer layer on the base fibers are layered such that the absorbent polymer expanding surfaces F are opposed to each other. When the gelled polymer expands from the layered absorbent cores 3A, 3B toward the opposed surfaces, a polymer layer 40 with a sufficient thickness is formed between the absorbent cores 3A and 3B, as schematically shown in FIG. 3 in particular. The gelled polymer layer 40 receives and disperses body pressure.

An amount of compression work of the absorber after polymer swelling is preferably 1.0 to 11.5 gf·cm/$cm^2$. If the amount of compression work is less than 1.0 gf·cm/$cm^2$, the absorber may exert no sufficient body pressure dispersion performance. An amount of compression work exceeding 11.5gf·cm/$cm^2$ would not be desirable in terms of shape retention of the absorber. A method for measuring an amount of compression work of the absorber in the present invention will be described below. First, a piece of absorber is cut in a size of 30 x 30 mm as a specimen. The specimen is put into a 50-mmΦ beaker, a physiological salt solution is added to the beaker, and then the specimen is swollen for 10 minutes. A usage amount of the physiological salt solution is 40 g or more per an internally existing polymer of 1 g. Then, the specimen is taken out of the beaker and placed on a horizontal plane. At that time, due care should be taken not so as to collapse the polymer layer. Next, the specimen is compressed at the center, and an amount of compression work of the absorber is measured by a compression tester. The measurement is made under compression by a weight bearing of 50 g with a 2$cm^2$-area disc (a speed of 0.1 cm/sec). The compression tester may be KES-G5 manufactured by Kato Tech Co., Ltd., for example.

In addition, the gelled polymer layer 40 is formed so as to expand toward the sheet-like fiber base material surfaces 30(F), and therefore is hardly subjected to constraint by the sheet-like fiber base materials 30. Therefore, owing to the presence of the gelled polymer layer 40, mutual movement of the opposed absorbent cores 3A, 3B in the shearing direction is not restricted, whereby the absorbent cores can move in the shearing direction.

The foregoing advantages bring about prominent effects of body pressure dispersion and decubitus ulcers prevention, which have not ever been provided by conventional absorbent articles.

In the illustrated embodiment, the absorbent cores form a two-layered structure for the purposes of providing ease of manufacture and assuring an appropriate thickness, but an arrangement of the absorbent cores is not limited to a two-layer structure.

In addition, there is no limitation to a thickness of the gelled polymer layer 40 after liquid absorption, but a preferred thickness of the same is about 5 to 20 mm.

The absorbent cores are desirably 10 to 60 weight % in ratio of polymer expanding amount after swelling, and preferably 35 to 45 weight %. A ratio of less than 10 weight % might lead to insufficient body pressure dispersion performance. If the ratio exceeds 60 weight %, displacement of the absorbent cores may become too large, which is undesirable in terms of shape retention of the absorber.

A method for measuring a polymer expanding amount ratio in the present invention will be described below. First, a piece of absorbent core is cut in a size of 30 x 30 mm as a specimen. The specimen is put into a beaker with an inner diameter of 50mmΦ, a physiological salt solution is added to the beaker, and then the specimen is swollen for 10 minutes. A usage amount of the physiological salt solution is 40 g or more per an internally existing polymer of 1 g. After that, the specimen is taken out of the beaker, and placed on a horizontal plane such that the polymer expanding surface is on topside, and then the specimen is weighed. At that time, due care should be taken not so as to collapse the expanding polymer on the polymer expanding surface. Then, the polymer layer is scraped off 10 times with a spatula from the polymer expanding surface not so as to crush the polymer expanding surface. The scraped specimen is weighed, and then a polymer expanding amount ratio is calculated in accordance with Equation 1 shown below.

[Equation 1] Polymer expanding amount ratio = (weight of scraped expanding polymer in the specimen/weight of the specimen after the 10-minute expansion) x 100

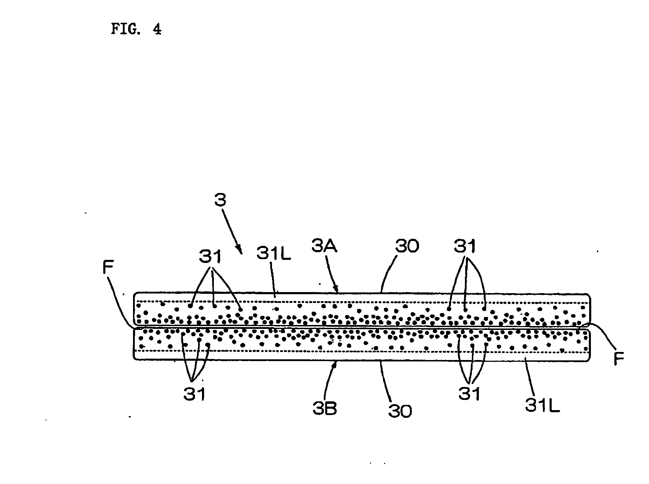

Meanwhile, in order to form the gelled polymer layer 40 on the sheet-like fiber base materials due to swelling by gelation after liquid absorption, the absorbent polymer 31 is scattered in the sheet-like fiber base materials 30 in such a manner that the absorbent polymer 31 is unevenly distributed with an increasing basis weight toward the absorbent polymer expanding surfaces F, for example as shown in FIG. 4, particularly in such a manner that a sufficient amount of absorbent polymer 31 is positioned in the vicinities of the absorbent polymer expanding surfaces F.

In addition, different kinds of polymers may be positioned from the absorbent polymer expanding surfaces F toward the opposite surfaces. For example, an absorbent polymer with high-absorbent, high-swelling, large-particle diameter properties or the like may be located on the absorbent polymer expanding surfaces F, and an absorbent polymer with lower-absorbent, lower-swelling, and lower-particle diameter properties or the like may be located on the opposite surfaces.

An amount of absorbent polymer 31 required for expansion can be decided as appropriate in consideration of a density of the sheet-like fiber base materials 30, inter-fiber gaps, and a swelling ratio of the absorbent polymer 31, and the like. As an example of a method for unevenly distributing the absorbent polymer in a thickness direction in the sheet-like fiber base materials 30, a large amount of adhesive may be dispersed on one surface of each of the sheet-like fiber base materials 30 so that a large amount of absorbent polymer 31 can be adhered to the one surface. As another example, the absorbent polymer 31 may be unevenly distributed by a sucking action. Alternatively, the absorbent cores 3A, 3B themselves may be formed by laminating layers with different polymer basis weights.

If using polymers of kinds listed below or other general absorbent polymers for use in this type of absorbent articles, it is preferred to form the absorbent cores each in a layered structure that has a low-basis weight layer with an absorbent polymer basis weight of 0 to 200 $g/m^2$ and a high-basis weight layer with an absorbent polymer basis weight of 150 to 400 $g/m^2$ which is located closer than the low-basis weight layer to the absorbent polymer expanding surface. Further, as in the example of an absorber shown in FIG. 4, it is preferred to provide a non-basis weight layer with an absorbent polymer basis weight of virtually 0 $g/m^2$ in such a manner as to be closer than the low-basis weight layer to the surface opposite to the polymer expanding surface, for the purpose of providing ease of expansion toward the desired surface F. In this arrangement, the fiber density of the non-basis weight layer is desirably higher than the two other layers.

Moreover, for preferably forming the gelled polymer layer 40 on the sheet-like fiber base materials 30, it is more effective to weaken an adhesion force between the fibers in the sheet-like fiber base materials 30 and the absorbent polymer 40 or reduce a size of an area of attachment between the two.

Accordingly, the absorbent polymer 31 attached to the fibers becomes prone to lose adhesion to the fibers after swelling due to liquid absorption. In addition, the absorbent polymer 31 swells and is apt to separate from the fibers only at regions other than minimal attachment regions attached to the fibers. As a result, the absorbent polymer 31 preferably swells toward the desired surface through inter-fiber gaps, thereby forming the gelled polymer layer 40.

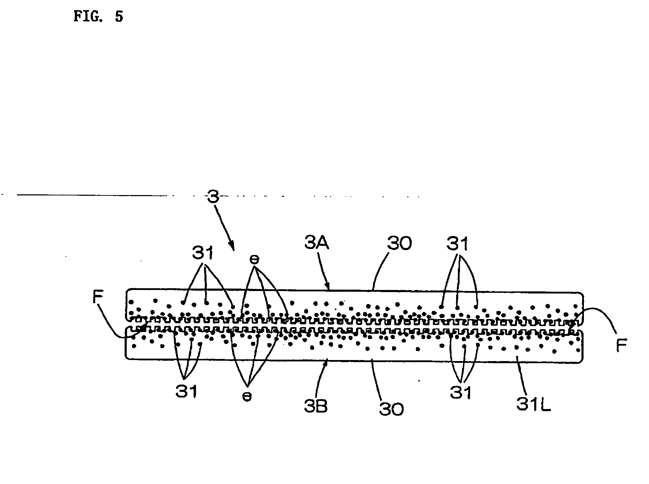

Further, in formation of the gelled polymer layer 40 on the sheet-like fiber base materials 30, the absorbent cores 3A, 3B are preferably subjected to embossing e on the absorbent polymer expanding surfaces F, as shown in the example of an absorber in FIG. 5, for instance.

By providing such embossing, the area of the expanding surface becomes larger in which an increased amount of polymer is thus exposed. Therefore, the polymer expands easily from inside the base material toward the desired surface. For example, when the sheet-like fiber base materials 30 are subjected to embossing, the absorbent polymer 31 can be unevenly distributed in the sheet-like fiber base materials 30 simply by dispersing the absorbent polymer 31 onto the embossed surfaces. This brings about an advantage that the absorbent cores in this embodiment can be easily manufactured.

In this embodiment, there is no limit to an embossing pattern. This kind of absorbers or absorbent cores can use any of conventional embossing patterns and embossing methods.

It is preferred to use a hot-melt adhesive to fix the absorbent polymer 31 to the fibers in the sheet-like fiber base materials 30. A hot-melt adhesive is excellent in handling performance and is capable of attaching the absorbent polymer 31 to the fibers by a weak binding force, whereby the absorbent polymer can be dispersed in such a manner as to expand reliably toward the surfaces of the fiber base materials after liquid absorption.

Such a hot-melt adhesive may preferably contain, as constituents, at least: a base polymer such as a styrene block copolymer, a polyurethane block copolymer, a polyester block copolymer, a polyamide block copolymer, or copolymer blend thereof adhesiveness-imparting components which are solid at ambient temperatures, such as a rosin resin, a polyterpene resin, a terpene phenol resin, a dicyclopentadiene petroleum resin, an aliphatic petroleum resin, an aromatic petroleum resin, a copolymer petroleum resin, an alicyclic petroleum resin, a xylene resin, and an elastomer; plastic components such as waxes; and an antioxidant.

In this embodiment, a ratio of fixation of polymer to the sheet-like fiber base materials in the absorbent core is desirably 45 to 95%, in particular desirably 80 to 95%. If the ratio is less than 45%, the polymer provides grain texture and decreases in tendency to form a desired polymer layer. In contrast, if the ratio exceeds 95%, the polymer also becomes less prone to form a desired polymer layer.

A method for measuring a ratio of polymer fixation in the present invention will be described below. First, an absorbent core with a cut or shaped size of 270 x 70 mm is prepared as a specimen, and the specimen is weighed. Then, the specimen is put into a 280 x 200 mm resin bag not so as to distort the shape thereof, and then the bag is sealed. Subsequently, the bag is vibrated 50 times at one time per second with an amplitude of 100 mm, thereby to cause the absorbent polymer to fall from the fiber base material. The fallen absorbent polymer is weighed, and then the fixation ratio of absorbent polymer is calculated in accordance with Equation 2 shown below.

$$\text{[Equation 2] Fixation ratio of absorbent polymer} = 100\% - \{(\text{weight of the fallen polymer/original weight of the specimen}) \times 100\}$$

Meanwhile, the sheet-like fiber base materials 30 constituting the absorbent cores 3A, 3B may use a nonwoven fabric sheet, a tow fiber aggregate, a synthetic fiber aggregate, accumulated pulp, or the like.

A tow fiber aggregate, a synthetic fiber aggregate, and an air-laid nonwoven fabric sheet formed by an air-laid method, have strong entanglements between fibers, possess excellent shape stability, and constitute an absorbent core which does not become loose or collapsed at a time of movement in the shearing direction.

Constitutional fibers of a synthetic fiber aggregate may use any of general synthetic fibers based on olefin such as nylon, polyethylene terephthalate, polyethylene, and polypropylene, polyester, and polyamide, or may use a resin film sheet cut in a fibrous form.

Meanwhile, if the sheet-like fiber base material uses a nonwoven fabric sheet, a preferred density thereof is 0.01 to 0.25 g/cm$^3$. By setting the density of the fiber base material within the range in the present invention, it is possible to preferably disperse the absorbent polymer and expand the absorbent polymer 31 through inter-fiber gaps.

In addition, the nonwoven fabric sheet preferably contains short fibers, more preferably hydrophilic crimped short fibers. Specific examples of a nonwoven fabric sheet include synthetic fibers based on olefin such as polyethylene or polypropylene, polyester, polyamide and the like; recycled fibers such as rayon or cupra; and natural fibers such as cotton, which are processed by an appropriate method such as an air-laid method, a spun-lace method, a spun-bonded method, a point-bonded method, a thermal-bonded method, a-melt-blown method, a needle-punched method, or the like.

An air-through nonwoven fabric is preferred in realizing a high air gap ratio due to its bulkiness, and in allowing a sufficient amount of absorbent polymer to be easily dispersed, thereby rapidly forming a polymer layer on the fiber base material. Particularly preferred is an air-through nonwoven fabric formed by mixed fibers of polyethylene/polypropylene.

In terms of body pressure dispersion, uneven absorbent polymer distribution, polymer layer formation, and the like, a particularly preferred nonwoven fabric sheet forming the sheet-like fiber base materials is an air-through nonwoven fabric sheet with a basis weight of 20 to 50 g/m$^2$ and a thickness of 0.4 to 10 mm, which contains short fibers with a fiber diameter of 2 to 20 dtex and a fiber length of 20 to 70 mm.

On the other hand, if the sheet-like fiber base material 30 uses accumulated pulp, there is no limitation to an original material for the accumulated pulp. Examples of such an original material include publicly known materials such as cotton pulp and synthetic pulp. In addition, there is no particular limitation to original pulp fibers. Examples of original fibers include cellulose fibers obtained from wood materials such as mechanical pulp, chemical pulp, and dissolving pulp; and artificial cellulose fibers such as rayon and acetate. However, wood materials for cellulose fibers are preferably originated from needle-leaved trees due to their longer fiber lengths than broad-leaved trees, in terms of functionality such as air gap formation and cost performance.

Meanwhile, the absorbent polymer 31 dispersed in the sheet-like fiber base material 30 has desirably a liquid absorption amount of 40 to 100 g/g measured by the tea bag method. If the liquid absorption amount of the absorbent polymer 31 exceeds 100 g/g, the gelled polymer layer 40 becomes too thick and increases a possibility of deteriorated wear comfort. In addition, too large a liquid absorption amount would be likely to cause excessive displacement of the absorbent cores 3A, 3B in a boundary therebetween, resulting in a higher possibility of a decreased fit. In contrast, if the degree of swelling is less than 40 g/g, there will arise a higher possibility where the gelled polymer layer 40 is insufficiently formed, which brings about no desired body pressure dispersion effect.

Measurement of a liquid absorption amount by the tea bag method will be described according to the following steps (1) to (6):

(1) An absorbent polymer of 0.5 g is put into a tea bag (made of 200-mesh nylon) to prepare a specimen.

(2) 100-cc physiological salt solution is put into a 200-cc beaker, and the specimen is soaked in the physiological salt solution for 30 minutes.

(3) The specimen is taken out of the physiological salt solution, and the tea bag is hung above the beaker to drain the specimen for five minutes. The draining is carried out at an ambient temperature ranging from 15 to 30 °C and at a humidity raging from 40 to 80%.

(4) The drained specimen is weighed.

(5) Another empty tea bag with no absorbent polymer is subjected to the foregoing steps (1) to (4), and the tea bag is weighed after draining.

(6) An amount of absorbed physiological salt solution per an absorbent polymer of 1g is calculated by Equation: ([weight of the drained specimen] - [weight of the drained tea bag])/0.5, and the calculated value is set as an absorbed liquid amount.

In addition, the absorbent polymer ranges desirably 0 to 10 mm in gel strength after swelling. If the gel strength of the absorbent polymer exceeds 10 mm, the polymer layer may be completely collapsed under body pressure, which provides no desired body pressure dispersion effect.

In the method for measuring a gel strength, an absorbent polymer of 2 g as a measurement specimen is put into a 200-cc beaker with an opening size of 67Φ, and a physiological salt solution of 60 cc is added to the beaker. The beaker is left for three minutes to allow the polymer to absorb the liquid. Then, a 500-g ball is gently placed and held for three minutes on the swelling surface of the absorbent polymer absorbing the liquid. After that, a sinking length of the ball is measured and set as a gel strength. The sinking length here refers to a distance from the swelling surface of the polymer to a lowermost part of the ball.

Further, the absorbent polymer is desirably 200 to 1,500 mpa·s in viscosity after swelling. If the viscosity is less than 200 mpa·s, the absorbent cores may not preferably move in the shearing direction. If the viscosity exceeds 1, 500 mpa·s, the absorbent cores may excessively move and thus may not fully hold the body of a wearer.

A method for measuring a viscosity in the present invention will be described below. First, a polymer of 1 g as a specimen is put into a container with an internal diameter of 50 mmΦ. A physiological salt solution of 40 g is added to the beaker, and the beaker is left as it is for 10 minutes to swell the absorbent polymer. Subsequently, a viscometer probe is inserted into the swollen polymer to cause vibrations for 10 minutes, and then a magnitude of a vertically acting interval resistance per unit area under the vibrations is measured. The viscometer may be VM-10A manufactured by CBC Co., Ltd.

Specifically, the absorbent polymer 31 can be selected from carboxymethyl cellulose, polyacrylic acid and salts thereof, a cross-linked product of acrylate polymer, a starch-acrylic acid grafting copolymer, a starch-acrylonitrile grafting copolymer hydrolysate, a cross-linked product of polyoxyethylene, a cross-linked product of carboxymethyl cellulose, a partially cross-linked product of water-swellable polymer such as polyethylene oxide or polyacrylamide, and a copolymer of isobutylene and maleic acid, and the like. The foregoing materials may be mixed with an antiblocking agent to suppress blocking in a product due to liquid absorption. The absorbent polymer may take any of various forms in the present invention, such as powder, particles, granulation, pellet, sol, suspension, gel, film, nonwoven fabric, and others, and preferred in particular is the form of particles. The absorbent polymer may be a granulated material or a pulverized material.

An average particle size of the absorbent polymer (measured by the screening method) can be appropriately decided in consideration of the density of the fiber base material or the like, and preferably is 150 to 500 μm, in particular preferably 200 to 400 μm. If the particle size is less than 150 μm, a polymer fixation ratio may be decreased. If the particle size exceeds 500 μm, it becomes difficult to disperse the polymer in the sheet-like fiber base materials.

Meanwhile, in the disposable diaper X1 of this embodiment, the absorber 3 has sheet-like dispersion members 50, 50 formed by nonwoven fabric sheets on both the top and back sides, as a particularly preferred example is shown in FIG. 2. On the top side of the absorber 3, the sheet-like dispersion member 50 may be positioned above or under the cover sheet.

Although the sheet-like dispersion members 50, 50 are provided on both the top and back sides of the absorber in the illustrated example, the sheet-like dispersion members 50, 50 may be on either of the sides. The sheet-like dispersion member is desirably formed of an air-through nonwoven fabric sheet. By providing such sheet-like dispersion members, body fluids such as urine or blood can be rapidly dispersed over the entire absorber, thereby achieving effective utilization of the entire absorbent cores. In particular, if a nonwoven fabric sheet, especially an air-through nonwoven fabric sheet is interposed between the absorber and the liquid permeable top sheet, the absorber is increased in degree of freedom of movement in the thickness direction, whereby body pressure can be more easily dispersed. In addition, the interposition of such a sheet makes it possible to prevent return of body fluids from the absorber and set an increased distance between the wearer's skin and the absorber, thereby providing more favorable wear comfort after liquid absorption.

Further, this embodiment has a pulp layer 60 formed by turning accumulated pulp into a sheet form on the back side of the absorber 3 under the sheet-like dispersion member 50. The pulp layer 60 accelerates body fluid absorption and increases a body fluid absorption capacity, thereby to reduce an unintended leakage of body fluids. The accumulated pulp may be the same as the accumulated pulp described above as being for use in the sheet-like fiber base materials.

In addition, there may be a sheet with properties of allowing body fluids to diffuse and permeate, called a second sheet, between the absorber 3 and the liquid permeable top sheet 1. A material for the second sheet only needs to have such properties of allowing body fluids to diffuse and permeate as stated above, and the material may be the same as that for the liquid permeable top sheet 1, for example. The second sheet is preferably more excellent in body fluid permeability than the liquid permeable top sheet, and in particular preferably the second sheet is formed of nonwoven fabric that is smaller in fiber density than the liquid permeable top sheet 1.

In the illustrated embodiment, the absorbent core 3A on the skin-contact side (the liquid permeable top sheet 1 side) and the absorbent core 3B on the non-skin-contact side (the liquid impermeable back sheet 2 side) are the same in thickness and size, but they may not be necessarily the same. For example, the absorbent core 3A on the skin-contact side (the liquid permeable top sheet 1 side) may be made smaller in front view than the absorbent core 3B on the non-skin-contact side (the liquid impermeable back sheet 2 side), or vice versa. The absorbent cores may have individual sizes and shapes as appropriate in consideration of the shape of the absorber fitted to the wearer's skin. The shapes of the absorbent cores may be adapted as appropriate to those of absorbers or absorbent cores used for known absorbent articles of this type such as disposable diapers.

Further, the absorbent cores 3A, 3B may be different in thickness, kind of the absorbent polymer used, kind and density of the sheet-like fiber base materials used, and the like. The absorbent cores may be provided in different arrangements within the scope of arrangement of the absorbent cores in the present invention described above.

<Second embodiment>

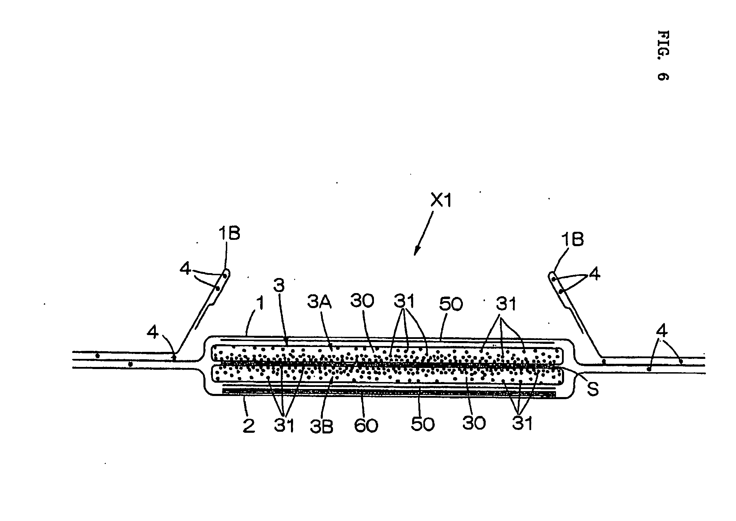

Next, a second embodiment will be described below. FIG. 6 shows the second embodiment. As apparent from the diagram, this embodiment has a sheet S of accumulated pulp or nonwoven fabric interposed between the adjacent absorbent cores. In respects other than the sheet S of accumulated pulp or nonwoven fabric, the second embodiment is identical to the first embodiment. Therefore, this embodiment is identical in front view to the first embodiment in FIG. 1. By interposing such an accumulated pulp or nonwoven fabric sheet between the absorbent cores, it is possible to adjust the absorber in solidity, disperse body fluids between the absorbent cores, and retain body fluids between the absorbent cores.

In addition, by interposing the sheet S of accumulated pulp or nonwoven fabric between the absorbent cores, upon formation of a polymer layer, the swollen polymer constituting the polymer layer attaches to the sheet S. This makes it possible to prevent the polymer from returning to the fiber base material and also prevent the formed polymer layer from disappearing or becoming thinner. This results in improved persistence of an effect of absorbent core movement and an effect of body pressure dispersion.

If the sheet S is a nonwoven fabric sheet that is excellent in body fluid dispersion and is the same in arrangement as the second sheet, it is possible to disperse body fluids between the absorbent cores, and spread and move the body fluids widely in the absorbent core on the non-skin-contact side. This effectively prevents an uneven polymer layer forming part of the absorbent core on the non-skin-contact side.

If the sheet S is a sheet excellent in body fluid retention such as accumulated pulp, it is possible to favorably enhance an effect of body fluid retention between the absorbent cores, thereby ensuring a sufficient time required for preferably forming a polymer layer between the adjacent absorbent cores.

In this embodiment, the sheet S may use the same fiber material as that of the sheet-like fiber base materials in the absorbent cores. However, the sheet S is preferably thinner and smaller in density than the sheet-like fiber base materials. The sheet S has preferably no absorbent polymer inside. A preferred thickness of the sheet S ranges specifically from about 0.5 to 1.0 mm. In addition, a preferred size of the sheet S is approximately identical to that of the absorbent core or slightly smaller than the same.

<Third embodiment>

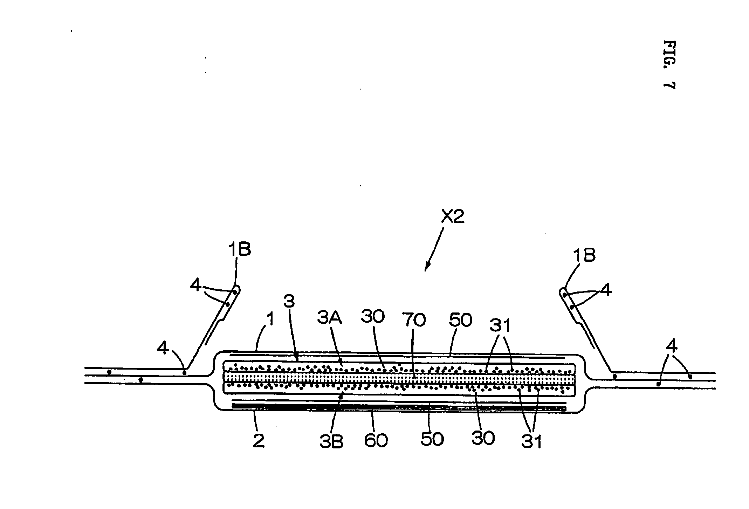

FIGs. 7 to 9 show a third embodiment. In the third embodiment, a movement auxiliary layer is provided between the adjacent absorbent cores to thereby facilitate relative and mutual movement of the absorbent cores in the shearing direction. In respects other than the absorbent cores and the movement auxiliary layer, the third embodiment is the same as the first embodiment. Therefore, the third embodiment is identical in front view to the first embodiment shown in FIG. 1.

FIG. 7 is a cross-section view showing characteristically a disposable diaper in this embodiment. As shown in FIG. 7, the disposable diaper in this embodiment has two absorbent cores 3A, 3B with a movement auxiliary layer 70 therebetween. The number of the absorbent cores is not limited to two, and thus three or more absorbent cores may be layered. The absorbent cores 3A, 3B in this embodiment have each a structure in which the absorbent polymer is scattered in the fiber base material, as in the first embodiment.

The fiber base material 30 in this embodiment may use a nonwoven fabric sheet, a tow fiber aggregate, a synthetic fiber aggregate, accumulated pulp, or the like, as described in relation to the first embodiment.

In using the absorbent polymer, this embodiment may be the same as the first embodiment, in a method for scattering the polymer in the fiber base material, a method for fixing the polymer, and a usage amount of the polymer.

The movement auxiliary layer 70 will be described in detail below. This layer is intended to reduce either a force equivalent to a static friction coefficient or a force equivalent to a dynamic friction coefficient between the absorbent cores at at least formation of a polymer layer. Preferably, even when no polymer layer is formed, this layer facilitates movement of the absorbent cores in the shearing direction. The force corresponding to a friction coefficient refers to, with the vertically layered absorbent cores, a constant force required for pulling and moving the upper absorbent core in the shearing direction according to the horizontal method specified by JIS P 8147.

The movement auxiliary layer 70 is formed by interposing a movement auxiliary material 71 mainly formed by a gas, a liquid, or particles or a mixture thereof between the absorbent cores, and is most preferably formed by particles or a mixture of particles and an appropriate amount of liquid, in terms of body fluid retention and shape stability.

Examples of a specific material suited for the movement auxiliary material 71 include air, polymer particles, gel, bead particles, granular urethane, synthetic sponge, natural sponge, lubricating polymers, porous silicon seat, silicon resin, silicon particles, and others.

The movement auxiliary layer 70 preferably ranges in thickness from about 5 to 20 mm, but is not limited to. In using any particles as a material, an average particle size may be designed as appropriate in consideration of the fiber density of the fiber base material 30 or the like. Similarly, the particle density of the movement auxiliary layer may also be designed as appropriate.

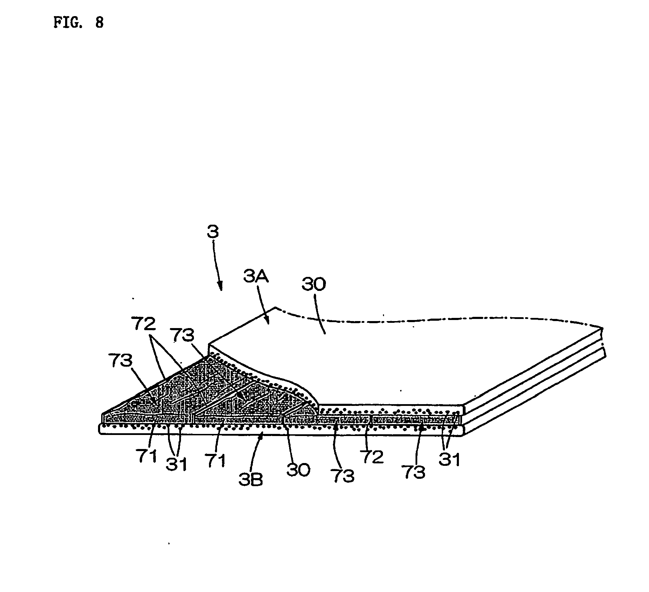

The movement auxiliary layer 70 may have an arrangement described below so as to prevent unintended outflow of the movement auxiliary material 71 from between the absorbent cores 3A, 3B and retain the movement auxiliary material 71 between the absorbent cores 3A, 3B. Specifically, as shown in FIG. 8, partitioning walls 72 are bridged between the adjacent absorbent cores 3A, 3B to divide the absorbent cores into a plurality of sections 73, and the movement auxiliary material 71 is charged into the partitioned sections 73, 73. The partitioning walls 72 may be formed by the same material as that of the liquid permeable top sheet, and can be attached to the absorbent cores 3A, 3B by a hot-melt adhesive or the like. The partitioning walls 72 may not be necessarily attached to the absorbent cores 3A, 3B, or may be attached to only one of the absorbent cores, 3A, for example. With such a structure, it is possible to prevent outflow of the movement auxiliary material 71 and also prevent excessive displacement of the absorbent cores 3A, 3B in the shearing direction.

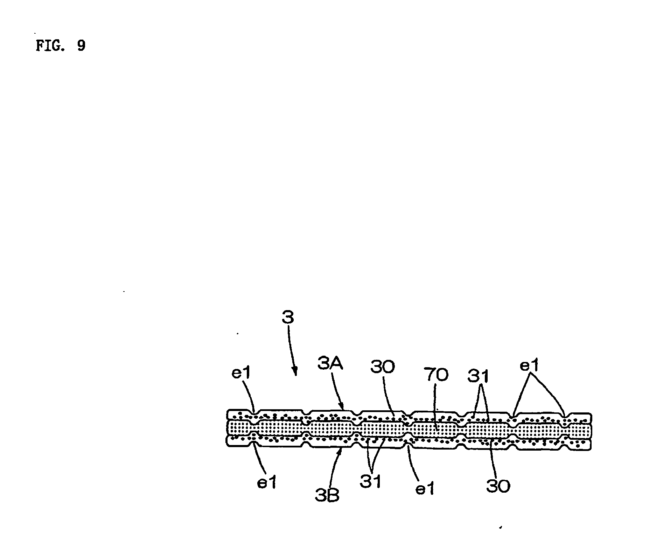

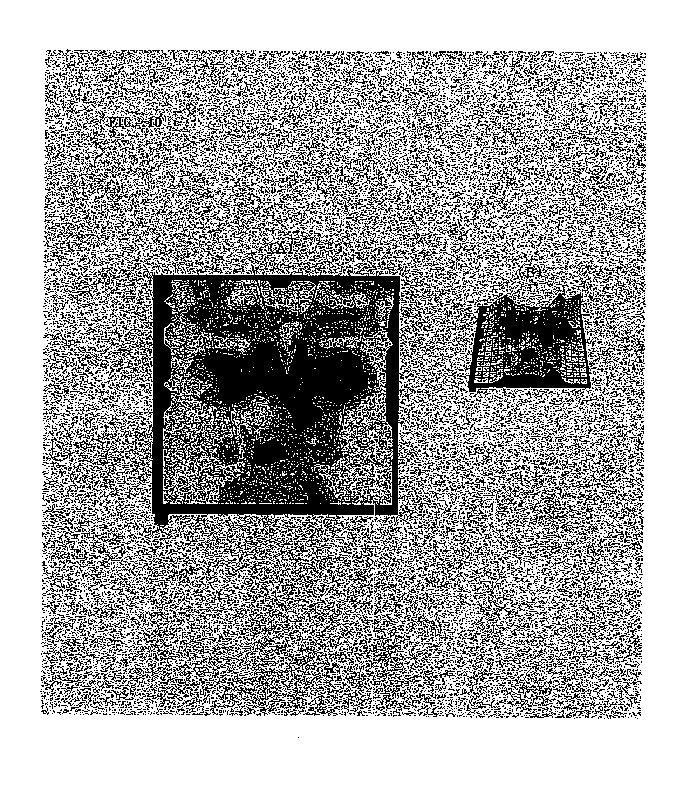

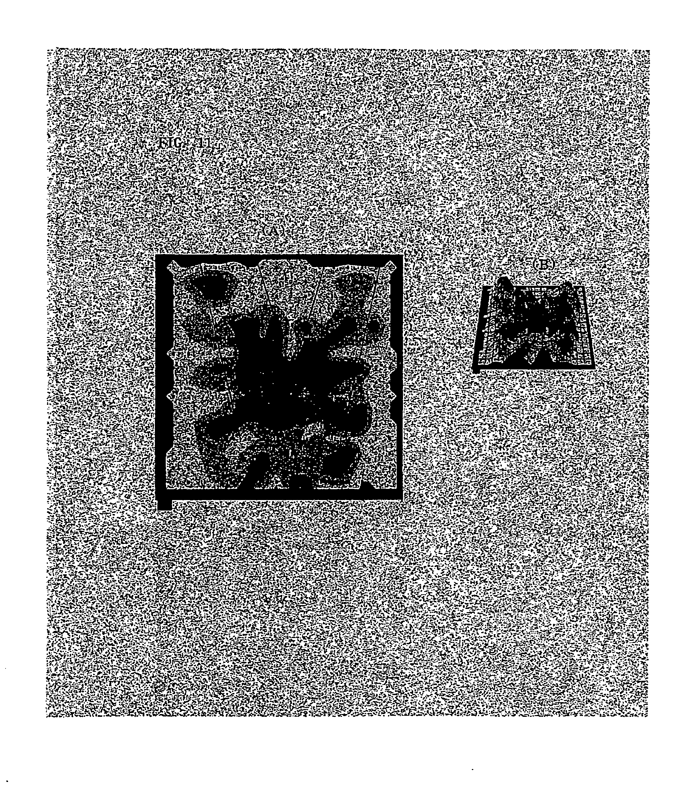

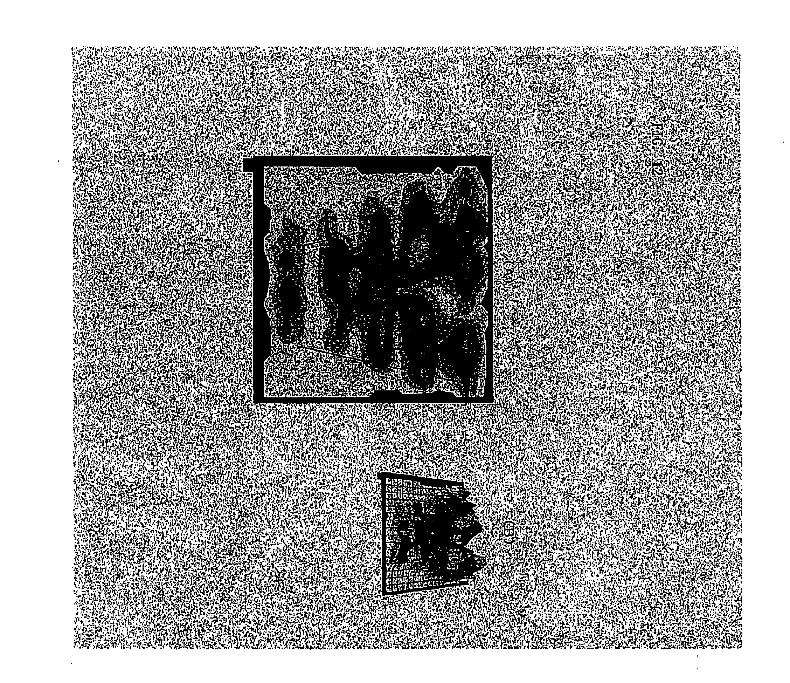

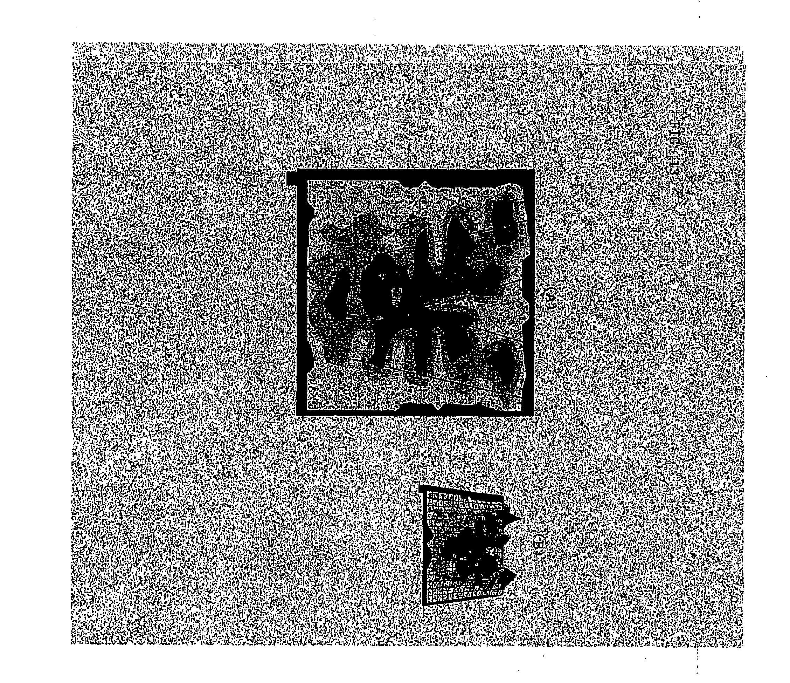

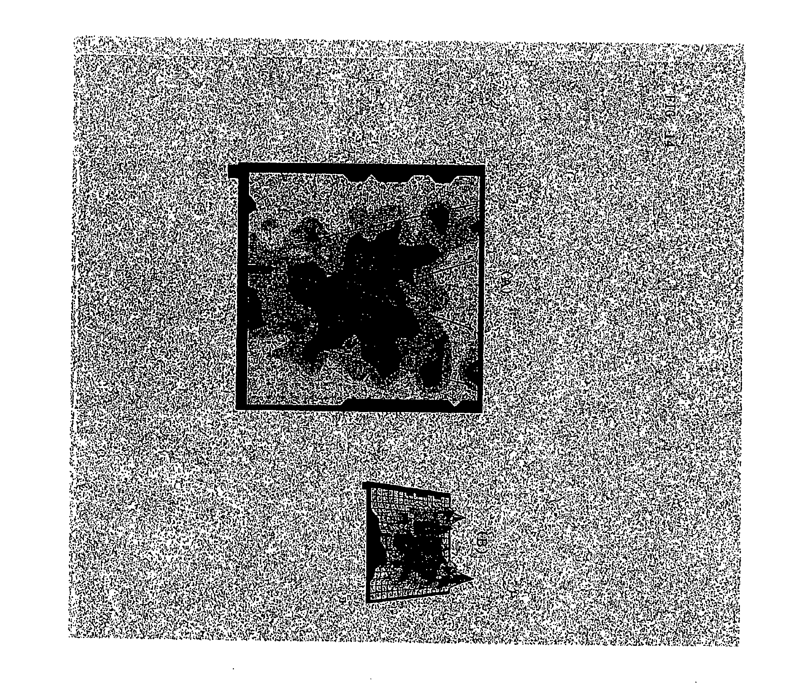

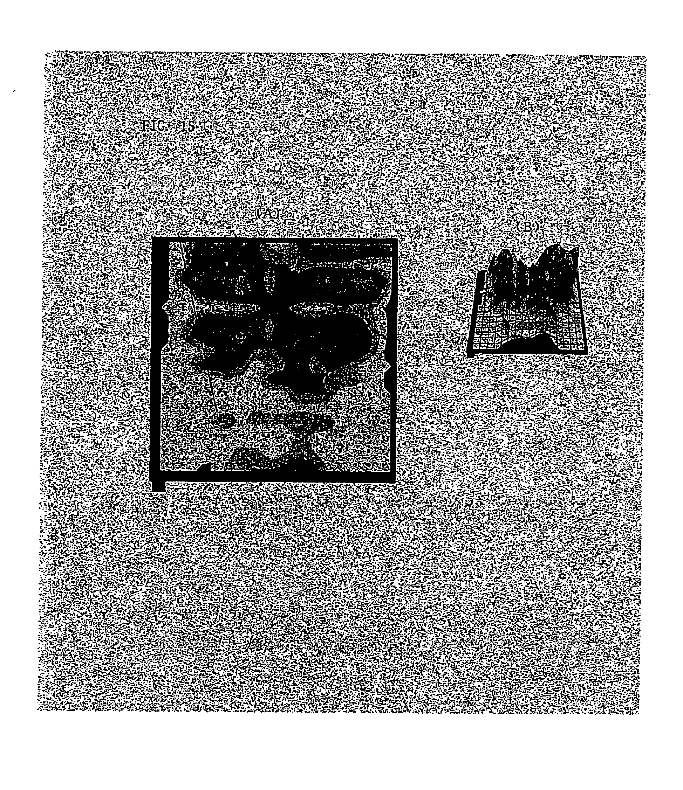

Meanwhile, in order to prevent unintended displacement of the absorbent cores 3A, 3B, the adjacent absorbent cores may have emboss portions e1,e1 so that the absorbent cores can be integrated as shown in FIG. 9. Providing such emboss portions increases temporarily a degree of attachment between the layers 3A, 3B, and 70, whereby displacement of the absorbent cores can be prevented when no intended pressure is applied to the skin-contact side or non-skin-contact side of the absorber, for example, when the absorbent cores are unused. That is, it is possible to fabricate a product in which the absorbent cores are not displaced unless a desired body pressure is applied. The absorber may be embossed for obtaining this advantage. Such embossing also acts preferably on the first embodiment.

In this embodiment, the absorbent core 3A on the skin-contact side (liquid permeable top sheet 1 side) and the absorbent core 3B on the non-skin-contact side (liquid impermeable back sheet 2 side) may also not be necessarily the same in thickness and size. The absorbent cores 3A, 3B may be different in kind of the absorbent polymer used, kind and density of the fiber base material used, and the like. The absorbent cores may take different arrangements within the scope of arrangement of the absorbent cores in the present invention as described above.

Example 1

Next, an example of the present invention and a conventional example were tested for body pressure dispersion. The test was carried out in such a manner that an examinee wearing a disposable diaper of each example was laid down on a body pressure dispersion bed over which a pressure sensor mat was placed, and body pressure was measured around the examinee's buttocks.

More specifically, first, the disposable diaper with polymer absorbing a liquid was attached to the examinee, the examinee was laid supine for one minute on the body pressure dispersion bed with the pressure sensor mat thereon, and then body pressure was measured around the examinee's buttocks by the pressure sensor mat.

The conventional disposable diaper used is a general type that has one absorbent core with high-absorbent polymer evenly scattered. In other aspects of the layered structure, the conventional diaper conforms to that shown in FIG. 2.

Next, the examinee's upper body was gradually raised from a lying posture using a body pressure dispersion bed's elevating function to cock up an angle of the pressure sensor mat. The examinee was held for five minutes in a position of being elevated at a 45-degree angle with reference to the examinee's waist. Then, the pressure sensor mat was used to measure pressure around the examinee's buttocks.

Further, the examinee was gradually laid down again by using a body pressure dispersion bed's reclining function to make the bed flat, and the examinee was held for one minute in a supine posture. Then, the pressure sensor mat was used to measure pressure around the examinee's buttocks.

Measurement machines used at the foregoing tests are a FSA body sensor mat manufactured by Takano Co., Ltd.; and a body pressure dispersion bed manufactured by Cape Co., Ltd.

The disposable diaper used of the present invention has the arrangement of the first embodiment in which a polymer layer is generated by liquid absorption, and a specific layered structure thereof is shown in FIG. 2. The sheet-like fiber base materials have mixed fibers of polyethylene/polypropylene with a basis weight of 40 g/m$^2$. The basis weight of the high-absorbent polymer is 164 g/m$^2$. The polymer fixation ratio is 43 weight %, the average particle diameter is 379 $\mu$m (measured by the screening method), and the liquid absorption amount measured by the tea bag method is 62 g/g.

FIGs. 10 to 15 show image data of measurement results of body pressure. FIG. 10 shows image data of the example of the present invention in the first supine posture; FIG. 11 shows image data of the example of the present invention in the 45-degree elevated posture; FIG. 12 shows image data of the example of the present invention in the second supine posture; FIG. 13 shows image data of the conventional example in the first supine posture; FIG. 14 shows image data of the conventional example in the 45-degree elevated posture; and FIG. 15 shows image data of the conventional example in the second supine posture.

In each of the diagrams, the left section (A) shows an isobaric colored map in which portions of the same color (the same density) fall within the same pressure range. The lines in the section (A) denote an outline of the examinee's body The right section (B) is a three-dimensional view of the section (A).

As understood from comparison between the diagrams, in the first supine posture, the examinee wearing the conventional diaper receives high pressure around the buttocks, and the pressure becomes lower with increasing distant from the buttocks (refer to FIG. 13(B)). In contrast, the examinee wearing the diaper of the present invention receives approximately even pressure from the buttocks through hip sides to legs, which means that body pressure is dispersed (refer to FIG. 10(B)).

In addition, in the 45-degree elevated posture, the examinee with the conventional diaper receives high pressure from the lower buttocks to thighs, but hardly receives pressure at the hip (refer to FIG. 14). On the other hand, the examinee with the diaper of the present invention receives high pressure from the buttocks to legs, and also receives pressure at the hip, which means that body pressure is distributed (refer to FIG. 11).

Further, in the second supine posture, the examinee with the conventional diaper is under pressure only at the buttocks, and hardly receives pressure at other body sites (refer to FIG. 15). In contrast, the examinee with the diaper of the present invention receives approximately even pressure from the buttocks through hip sides to legs, which means that body pressure is distributed (refer to FIG. 12).

As a result, it has been found that the example of the present invention effectively caused body pressure dispersion, as compared with the conventional example.

Example 2

Next, disposable diapers were tested for usability with absorbers of the present invention and absorbers of comparative examples. In addition, the absorbers used in the tested diapers were evaluated for ease of movement in the shearing direction between absorbent cores positioned on the skin-contact side and the non-skin-contact side.

The absorbers in examples 1 to 5 of the present invention have each a structure in which absorbent cores are directly opposed to each other. The absorber in example 6 of the present invention has a structure in which a pulp layer is interposed between absorbent cores and is not fixed to the absorbent cores. The absorber in comparative example 1 has a structure in which absorbent cores wrapped by crepe paper are layered (the absorbent cores are adhered to the crepe paper). The absorber in comparative example 2 has a structure with one absorbent core.

Table 1 below shows test results and describes constituent materials of the absorbers in the foregoing examples and comparative examples. Used for the tests are fastening-type disposable diapers of a general shape in which an absorber is interposed between a liquid permeable top sheet and a liquid impermeable back sheet.

<Tensile force (ease of movement in the shearing direction)>

Each absorbent core constituting the non-skin-contact side was fixed on a horizontal plane, and each absorbent core constituting the skin-contact side was laid on the former absorbent core. Then, a 500-g cylindrical spindle with a diameter of 5.5 cm was placed on the skin-contact-side absorbent core. In this situation, the skin-contact-side absorbent core was tensed in the horizontal direction at a rate of 10 mm/min by a tensile tester machine (TENSILON manufactured by Orientec Co., Ltd.) for measurement of a tensile force. Since there was the need for obtaining measurements after liquid absorption, the specimens had been caused to absorb a physiological salt solution of 40 g per an absorbent polymer of 1 g.

The absorbent cores and the pulp layers as specimens were 100 x 250 mm in size and were tensed in the longitudinal direction.

<Sense of use>

For making assessments as to sense of use, diapers with the absorbers in the foregoing examples and comparative examples were subjected to sensory evaluation in which 30 examinees actually worn the diapers and assessed their perceptions about sense of use. Evaluations were made on a criterion that "the absorber can move in accordance with the movement of a wearer's buttocks with hardly a feeling of being rubbed". The diapers were rated for sense of use as excellent (◎) with 25 to 30 examinees' favorable assessments, as good (○) with 10 to 25 examinees' favorable assessments, or as poor (×) with less than 10 examinees' favorable assessments.

[Table 1]

| | Constituent | | | | | | | Tensile force | Sense of use |
|---|---|---|---|---|---|---|---|---|---|
| | Top layer-side absorbent core (the skin-contact side) | | | Intermediate layer (movement auxiliary layer) | Back layer-side absorbent core (non-skin-contact side) | | | | |
| | Fiber base material (type) | Base material fiber density (g/cm$^3$) | Polymer basis weight | Sheet density (g/cm$^3$) | Fiber base material (type) | Base material fiber density (g/cm$^3$) | Polymer basis weight | | |
| Example1 | Air-through nonwoven fabric | 0.008 | 160 | - | Air-through nonwoven fabric | 0.008 | 160 | 0.68 | ◎ |
| Example2 | Air-through nonwoven fabric | 0.008 | 160 | - | Air-through nonwoven fabric | 0.008 | 160 | 0.55 | ◎ |
| Example3 | Air-through nonwoven fabric | 0.04 | 200 | - | Air-through nonwoven fabric | 0.04 | 200 | 0.55 | ◎ |
| Example4 | Air-through nonwoven fabric | 0.008 | 0 | - | Air-through nonwoven fabric | 0.01 | 0 | 0.72 | ◎ |
| Example5 | Pulp | 0.03 | 160 | - | Air-through nonwoven fabric | 0.01 | 164 | 0.90 | ○ |
| Example6 | Air-through nonwoven fabric | 0.04 | 160 | Pulp (0.02) | Air-through nonwoven fabric | 0.008 | 160 | 0.80 | ◎ |
| Comparative example 1 | Pulp | 0.03 | 160 | - | Pulp | 0.03 | 164 | 2.25 | × |
| Comparative example 2 | Air-through nonwoven fabric | 0.016 | 320 | - | - | -- | - | - | × |

<Test results>

As shown in Table 1, it has been revealed that no movement of the absorbers was felt at all on comparative examples 1 and 2, whereas movement of the absorber in accordance with movement of the examinee's buttocks was felt on the disposable diaper with the absorber of the present invention. In addition, the example of the present invention also enjoyed favorable ratings for ease of movement of the absorbent cores in the shearing direction, which is recognized to have correlation with sense of use.

It is conceived that the examples of the present invention achieved favorable results owing to the polymer layer between the absorbent cores.

The inventors of the present invention found that, if an absorbent core is determined to have a tensile force of 2.0 or more at a test for ease of movement in the shearing direction, the absorbent core hardly produces a feeling of movement when being incorporated in a disposable diaper. The test results shown in Table 1 prove the findings.

As stated above, the absorbent articles of the present invention are effective in body pressure dispersion, particularly body pressure dispersion with the absorber absorbing body fluids or the like, and effective in prevention of decubitus ulcers.

Industrial Applicability

The present invention is applicable to absorbent articles for use in body sites to which pressure is applied, such as underpants-type disposable diapers, fastening-type disposable diapers, sanitary napkins, absorbent pads, urine pads, and others, for example.

Brief Description of Drawings

FIG. 1 is a plane view of a fastening-type disposable diaper of a first embodiment;
FIG. 2 is a cross-section view of FIG. 1 as taken along line II-II;
FIG. 3 is a schematic diagram showing a cross section of an absorber of the first embodiment at formation of a polymer layer;
FIG. 4 is a schematic diagram showing a cross section of another absorber of the first embodiment;
FIG. 5 is a schematic diagram showing a cross section of still another absorber of the first embodiment;
FIG. 6 is a cross-section view of a fastening-type disposable diaper of a second embodiment;
FIG. 7 is a cross-section view of a fastening-type disposable diaper of a third embodiment;
FIG. 8 is a schematic diagram of an absorber in another absorbent core of the third embodiment;
FIG. 9 is a schematic diagram showing an absorber in still another absorbent core of the third embodiment;
FIG. 10 shows body pressure dispersion measurement image data of an example of the present invention in a first supine position;
FIG. 11 shows body pressure dispersion measurement image data of an example of the present invention in a 45-degree elevated position;
FIG. 12 shows body pressure dispersion measurement image data of an embodiment of the present invention in a second supine position;
FIG. 13 shows body pressure dispersion measurement image data of a conventional example in a first supine position;
FIG: 14 shows body pressure dispersion measurement image data of a conventional example in a 45-degree elevated position; and
FIG. 15 presents body pressure dispersion measurement image data of the conventional embodiment in a second supine position.

Brief Description of Numerals

1...Liquid permeable top sheet, 1B...Standing cuffs, 2...Liquid impermeable back sheet, 3...Absorber, 3A, 3B...Absorbent core, 6...Fastening tape, 7...Engagement section, 30...Sheet-like fiber base material, 31...Absorbent polymer 40...Gelled polymer layer, 50...Sheet-like dispersion member, 60...Pulp layer, 70...Movement auxiliary layer, 71...Movement auxiliary material, 72...Partitioning wall, 73...Section, e...Emboss, F...Absorbent polymer expanding side, X1...Fastening-type disposable diaper

**Claims**

**1.** An absorbent article having an absorber interposed between a liquid permeable top sheet and a liquid impermeable back sheet, wherein
the absorber has a layered structure in which absorbent cores each including at least a fiber base material and an absorbent polymer as constitutional elements are layered,
the absorbent polymer is scattered in the fiber base material in such a manner that the polymer swells by gelation due to liquid absorption and expands up to one surface of the fiber base material through inter-fiber gaps, thereby to form a polymer layer on the fiber base material,
absorbent polymer expanding surfaces of adjacent absorbent cores are opposed to each other, and
at least by formation of the polymer layer, the adjacent absorbent cores can move mutually in a shearing direction with the polymer layer therebetween.

**2.** The absorbent article according to Claim 1, wherein the fiber base material is a tow fiber aggregate, an air-laid nonwoven fabric, or a synthetic fiber aggregate.

**3.** The absorbent article according to Claim 1, wherein the absorbent polymer is 150 to 500 $\mu$m in average particle diameter, and
the fiber base material is an air-through nonwoven fabric with a basis weight of 20 to 50 g/m$^2$ and a thickness of 0.4 to 10 mm, which is formed by short fibers with a fiber diameter of 2 to 20 dtex and a fiber length of 20 to 70 mm.

**4.** The absorbent article according to any one of Claims 1 to 3, wherein the absorbent cores have the absorbent polymer unevenly distributed in such a manner that a basis weight thereof becomes larger with increasing proximity to the absorbent polymer expanding surfaces.

**5.** The absorbent article according to any one of Claims 1 to 4, comprising a movement auxiliary layer in which a movement auxiliary material formed by a gas, a liquid or particles, or a mixture thereof, is interposed between the adjacent absorbent cores.

**6.** The absorbent article according to Claim 5, wherein the movement auxiliary layer is formed in such a manner that a movement auxiliary material is charged into a plurality of sections divided by partitioning walls bridged between the adjacent absorbent cores.

**7.** The absorbent article according to any one of Claims 1 to 5, wherein the adjacent absorbent cores have a sheet formed of nonwoven fabric or accumulated pulp interposed therebetween.


**Patentansprüche**

**1.** Absorbierender Artikel mit einem Absorber, der zwischen einer flüssigkeitsdurchlässigen Deckschicht und einer flüssigkeitsundurchlässigen rückseitigen Schicht angeordnet ist,
wobei der Absorber eine geschichtete Struktur aufweist, in der absorbierende Kerne, die jeweils wenigstens ein Faserbasismaterial und ein absorbierendes Polymer umfassen, als grundlegende Elemente geschichtet sind,
wobei das absorbierende Polymer in dem Fiberbasismaterial auf so eine Weise verstreut ist, dass das Polymer durch Gelation infolge der Flüssigkeitsabsorption anschwillt und bis zu einer Oberfläche des Fiberbasismaterials durch Lücken zwischen den Fasern expandiert, um dadurch eine Polymerschicht auf dem Fiberbasismaterial zu bilden,
wobei expandierende Oberflächen aus absorbierendem Polymer von benachbarten absorbierenden Kernen einander gegenüberliegend sind und
zumindest bei Bildung der Polymerschicht die benachbarten absorbierenden Kerne sich gegenseitig in einer Scherrichtung mit den Polymerschichten dazwischen bewegen können.

**2.** Absorbierender Artikel nach Anspruch 1, wobei die Fiberbasisschicht ein Tow-Faseraggregat, ein Airlaid-Vliesstoff oder nicht verwebter Stoff oder ein synthetisches Faseraggregat ist.

**3.** Absorbierender Artikel nach Anspruch 1, wobei das absorbierende Polymer einen Durchschnittspartikeldurchmesser von 150 bis 500 $\mu$m hat und
sas Faserbasismaterial ein Durchluft-Vliesstoff oder ein mit Luft verfestigter Vliesstoff, mit einem Basisgewicht von 20-50 g/m² und einer Dicke von 0,4 bis 10 mm ist, der durch kurze Fasern mit einem Faserdurchmesser von 2 bis 20 dtex und einer Faserlänge von 20 bis 70 mm gebildet ist.

4.  Absorbierender Artikel nach einem der Ansprüche 1 bis 3, wobei die absorbierenden Kerne das absorbierende Polymer auf so eine Weise ungleichmäßig verteilt haben, dass ihr Basisgewicht mit steigender Nähe zu den expandierenden Oberflächen aus absorbierendem Polymer größer wird.

5.  Absorbierender Artikel nach einem der Ansprüche 1 bis 4, wobei er eine Bewegungshilfsschicht aufweist, in der ein Bewegungshilfsmaterial zwischen den benachbarten absorbierenden Kernen angeordnet ist, die durch ein Gas, eine Flüssigkeit oder Partikel oder eine Mischung derselben gebildet ist.

6.  Absorbierender Artikel nach Anspruch 5, wobei die Bewegungshilfsschicht auf so eine Weise gebildet ist, dass ein Bewegungshilfsmaterial in eine Vielzahl von Abschnitten geladen ist, die durch Trennwände aufgeteilt sind, die zwischen den benachbarten absorbierenden Kernen überbrückt werden.

7.  Absorbierender Artikel nach einem der Ansprüche 1 bis 5, wobei die benachbarten absorbierenden Kerne zwischen sich eine Schicht angeordnet haben, die aus Vliesstoff oder akkumuliertem Zellstoff gebildet ist.

**Revendications**

1.  Article absorbant ayant un absorbeur interposé entre une feuille supérieure perméable au liquide et une feuille inférieure imperméable au liquide, dans lequel
l'absorbeur a une structure stratifiée dans laquelle sont superposées des âmes absorbantes incluant chacune au moins un matériau à base de fibres et un polymère absorbant en tant qu'éléments constitutifs,
le polymère absorbant est dispersé dans le matériau à base de fibres de telle manière que le polymère gonfle par gélification en raison de l'absorption de liquide et s'expanse jusqu'à une surface du matériau à base de fibres au travers des ouvertures entre fibres, formant ainsi une couche polymérique sur le matériau à base de fibres,
les surfaces d'expansion du polymère absorbant des âmes absorbantes adjacentes sont opposées l'une à l'autre, et au moins grâce à la formation de la couche polymérique, les âmes absorbantes adjacentes peuvent se déplacer l'une par rapport à l'autre dans une direction de cisaillement avec la couche polymérique intercalées entre elles.

2.  Article absorbant selon la revendication 1, dans lequel le matériau à base de fibres est un agrégat de fibres d'étoupe, un tissu non tissé obtenu par voie aérodynamique, ou un agrégat de fibres synthétiques.

3.  Article absorbant selon la revendication 1, dans lequel le polymère absorbant a un diamètre moyen de particule situé entre 150 et 500 $\mu$m, et
le matériau à base de fibres est un tissu non tissé perméable à l'air avec une masse surfacique de 20 à 50 g/m² et une épaisseur de 0,4 à 10 mm, lequel est formé par des fibres courtes avec un diamètre de fibre de 2 à 20 décitex et une longueur de fibre de 20 à 70 mm.

4.  Article absorbant selon l'une des revendications 1 à 3, dans lequel les âmes absorbantes ont le polymère absorbant distribué de manière inégale de telle manière que sa surface massique augmente avec l'augmentation de la proximité des surfaces d'expansion du polymère absorbant.

5.  Article absorbant selon l'une des revendications 1 à 4, comprenant une couche auxiliaire de mouvement dans laquelle est interposé entre les âmes absorbantes adjacentes un matériau auxiliaire de mouvement formé par un gaz, un liquide ou des particules, ou un mélange de ces éléments.

6.  Article absorbant selon la revendication 5, dans lequel le matériau auxiliaire de mouvement est formé de telle manière qu'un matériau auxiliaire de mouvement est chargé dans une pluralité de sections séparées par des parois de partitionnement s'étendant entre les âmes absorbantes adjacentes.

7.  Article absorbant selon l'une des revendications 1 à 5, dans lequel les âmes absorbantes adjacentes ont une feuille formée d'un tissu non tissé ou d'une pâte accumulée et interposée entre elles.

FIG. 1

FIG. 2

FIG. 3

FIG. 4

FIG. 5

FIG. 6

FIG. 7

FIG. 8

FIG. 9

FIG. 10

(A)

(B)

FIG. 11

(A)

(B)

FIG. 12

(A)　　　　　(B)

FIG. 113

(A)

(B)

EP 2 111 835 B1

FIG. 14

(A)

(B)

31

FIG. 15

(A)

(B)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006102479 A